# EUROPEAN PATENT APPLICATION

(11) **EP 1 086 670 A2**
(43) Date of publication of application: **28.03.2001**
(21) Application number: 00305530.8
(22) Date of filing: 30.06.2000
(51) Int. Cl.: A61F 5/01

(54) **Adjustable orthopaedic brace**

(30) Priority: 27.09.1999 US 156241
(71) Applicant: DEPUY ORTHOPAEDICS, INC., Warsaw, Indiana 46581 (US)
(72) Inventor: ENZERINK, Robert-Jan, Davis CA 95219 (US)
(74) Representative: Belcher, Simon James

(57) **Abstract**

An orthopaedic brace includes an upper strut pivotally connected with a lower strut by a range of motion hinge. The range of motion hinge can be axially actuated to adjust or to set the angular range of motion of the hinge and therefore of the brace. A stop pin carried by an adjustment knob of the hinge assembly can be positioned in any of a plurality of settings corresponding to various ranges of motion for the hinge. The stop pin/adjustment knob is axially and rotatably movable to set a range of flexion and/or extension motion.

## Description

The present invention relates to orthopaedic braces and, more particularly, to a range of motion hinge adaptable to orthopaedic braces used in stabilizing a joint after injury or invasive surgery.

In order to ensure the proper healing of a human joint after an injury or invasive surgery, it is often desirable to limit the pivotal motion of the human joint to a predetermined angular range between full extension and full flexion. The pivotal motion may be limited by a range of motion hinge disposed between an upper strut and a lower strut. Orthopaedic braces of this general type, including information relating to range of motion braces, and how and why such equipment is used, are disclosed in US-4531515, US-4817588, US-5052379, US-5292303, US-5409449 and US-5817040.

It is well known that the range of motion braces described in the aforementioned patents suffer various problems, shortcomings and disadvantages. Most notably, the range of motion hinges tend to be difficult to operate, manipulate and adjust; to be bulky; to have high profiles that tend to snag on furniture, clothes and the like; to have complicated and hard to read adjustment mechanisms; and to have loose parts.

The present invention provides a range of motion hinge for an orthopaedic brace that includes an axially actuated range of motion setting assembly.

In one form, the present invention provides an orthopaedic joint brace that includes a first strut, a second strut, and a hinge assembly pivotally coupling the first strut with the second strut and defining an axis of rotation. A range of motion of the hinge assembly can be set selectively by an axially actuated stop.

In another form, the present invention provides an orthopaedic brace that includes an upper strut, a lower strut, and a hinge assembly disposed between the upper and lower struts. The hinge assembly provides for motion or movement of one of the struts about an axis corresponding to a joint axis. The hinge assembly can be set in a plurality of positions corresponding to a plurality of ranges of motion of the struts about the axis. The hinge assembly includes an axially actuated and rotatably mounted adjuster operable to selectively set the range of motion of the hinge assembly.

In yet another form, the present invention provides an orthopaedic brace that includes an upper strut, a lower strut, a hinge assembly disposed between the struts and providing for movement of one of the struts about an axis corresponding to a joint axis, and an adjustment knob carried on the hinge assembly. The hinge assembly includes opposing plates, each opposing plate containing a pivot aperture and a plurality of circumferential apertures forming pairs of retaining apertures. Each pair of retaining apertures corresponds to a range of motion of the struts about the axis. The opposing plates are rigidly connected to one of the upper and lower struts, and pivotally retain the other strut between them. The adjustment knob includes a retaining pin for engagement in a selective one of pairs of retaining apertures to select a range of motion of the struts about the axis.

Accordingly, the present invention improves upon the prior art by providing a low-profile, range of motion hinge that is easily adjustable by the wearer. More specifically, the improved range of motion hinge may be adjusted without the use of tools, and it is smaller in diameter, is lighter in weight, and is lower in profile in comparison to the prior art. This cost-effective range of motion hinge allows the selective restriction of range of motion in both extension and flexion through axially actuated stops in an enclosed system of locking pins and stop apertures.

The present invention will now be described by way of example with reference to the accompanying drawings, in which:
FIG. 1 is a side perspective view of an adjustable, motion-restraining knee brace containing an improved range of motion hinge assembly that embodies principles of the present invention showing the brace operatively connected to a human leg;
FIG. 2 is a side elevational view depicting the range of motion hinge of FIG. 1 showing an arbitrary degree of flexion and, in phantom, a full degree of extension;
FIG. 3 is an exploded, perspective view of the range of motion hinge assembly of FIG. 2; and
FIGS. 4A and 4B are cross-sectional views through the range of motion hinge assembly taken along line 4-4.

In the drawings, corresponding reference characters indicate corresponding parts throughout the several views.

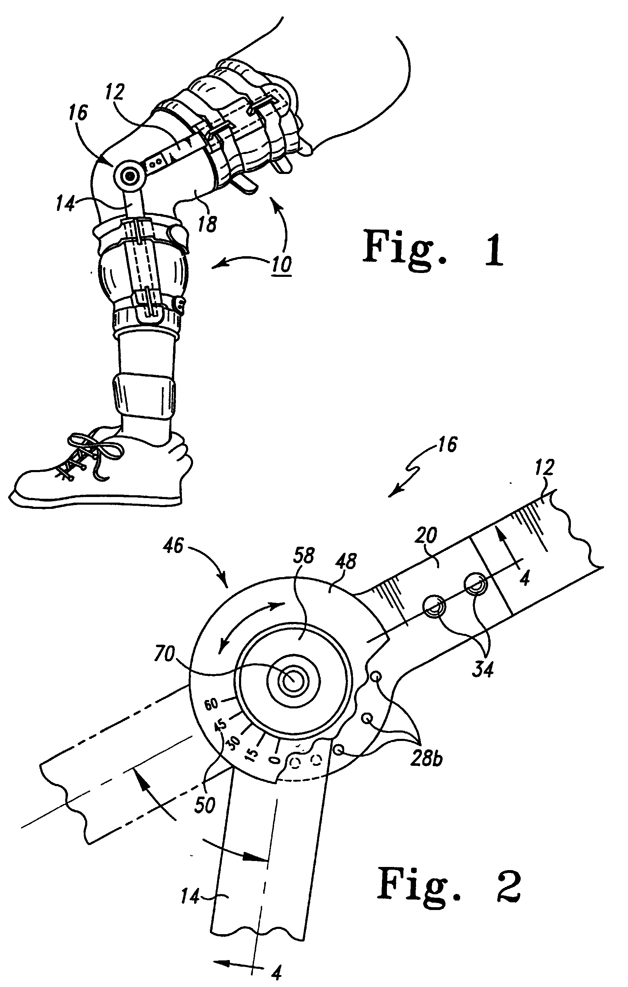

Referring to the drawings, Fig. 1 shows an orthopaedic brace 10 which is adapted to fit on a human limb in order to limit the range of motion of a human joint. While it is expected that this improved orthopaedic brace is adaptable to any of the joints of the human body, such as an elbow, an ankle or a hip, it will be described herein as used to aid the healing of the knee joint. Consequently, Fig. 1 shows the orthopaedic brace operatively connected to a human leg 18 through conventional means. Orthopaedic brace 10 includes an upper strut 12, a lower strut 14, and an improved range of motion hinge assembly 16 disposed between the upper strut 12 and the lower strut 14 while not shown, the orthopaedic brace preferably includes a like hinge and strut assembly on the opposite side thereof (of the leg) which cannot be seen in the figure.

Referring to Fig. 2, the improved range of motion hinge assembly 16 is shown in an arbitrary degree of flexion and, in phantom, in a full degree of extension with respect to the upper strut 12 and the lower strut 14. Also, shown is a release-and-adjustment knob assembly 46. The knob or adjustment assembly 46, has at least one movable bezel 48 with an easily readable, graded degree index scale 50. The bezel 48 is particularly rotatably movable about a centre or hinge pivot pin 70. The graded degree scale 50 shows the arcuate range of motion of the struts 12 and 14 relative to one another or the amount of rotatable movement allowed by the hinge assembly 16. Although not shown, the knob assembly 46 may, as will be described subsequently, also be adapted to include at least a second movable bezel that operates independently of the first bezel 48. The depicted degree index scale 50 shows, as an example, an included angle of 60 degrees at 15 degree intervals. It will be appreciated that the incremental angles and the maximum angles of extension and flexion may vary.

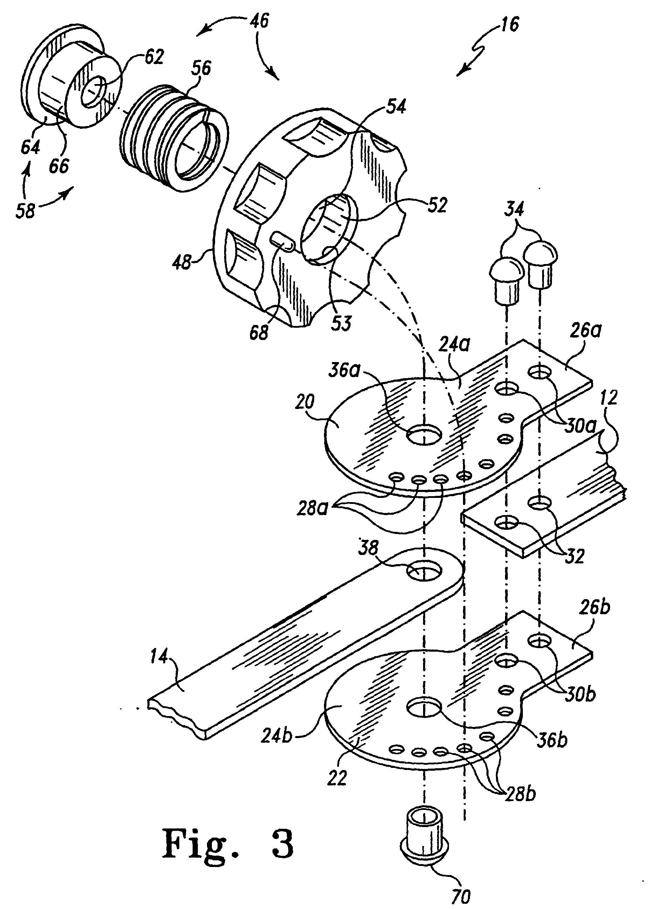

Fig. 3 further details the knob assembly 46. The bezel 48 defines at its centre a cylindrical chamber 52 adapted to receive a spring retainer member 58. At the centre of the chamber 52 is circular opening 54, of smaller radius than the chamber 52, formed the rest of the way through the bezel 48 and the bottom of the chamber 52. The spring retainer member 58 has a flanged-lipped top portion 64 and a cylindrical, elongated body portion 66. A circular opening 62 runs through the centre of the spring retainer member 58. The spring retainer member 58 is adapted to receive about its body portion 66 a spring or like component 56, which slides onto or around the body portion 66 and is retained between the flange portion 64 and the bottom 53 of the chamber 52. The bezel 48 is fitted with a stop pin 68. Although not depicted, additional stop pins could also be attached to the bezel 48.

Fig. 3 also details further portions of the hinge assembly 16, that pivotally interconnects the upper strut 12 and the lower strut 14. The hinge assembly 16 includes a pair of outer and inner opposing base plates 20 and 22, which may be identically configured. Each of the opposing base plates 20 and 22 has a generally circular body portion 24a and 24b respectively from which a generally rectangular connecting tab portion 26a and 26b respectively tangentially extends. Formed through the periphery of the body portions 24a and 24b are an aligned plurality of circumferentially spaced stop apertures 28a and 28 respectively. The apertures 28a and 28b are adapted to receive the stop pin 68. Also formed through the body portions 24a and 24b, at their centres, is a circular opening 36a and 36b respectively, adapted to receive the hinge pivot rivet 70, the function of which subsequently will be described.

A central end portion of the upper strut 12 is sandwiched and retained between the opposing base plates 20 and 22, as shown in Fig. 3, and anchored to the respective connecting tabs 26a and 26b of the opposing base plates 20 and 22 by means of two rivets 34. The two rivets 34 are extended down into aligned openings 30a and 30b formed through the respective tabs 26a and 26b, and through the aligned openings 32 in the upper strut 12. The central end portion of the lower strut 14 is also sandwiched and retained, pivotally, between the opposing base plates 20 and 22. The central end portion of the strut 14 has a circular opening 38 extending through it, which is aligned with the central circular openings 36a and 36b respectively formed through the circular body portions 24a and 24b of the respective base plates 20 and 22. The hinge pivot rivet 70 pivotally connects the components of the hinge assembly 16 and the accompanying adjustment assembly 46 by extending down sequentially through the bezel chamber opening 54, the retainer opening 62, the outer plate 20 body opening 36a, the lower strut opening 38, and the inner plate 22 body opening 36b.

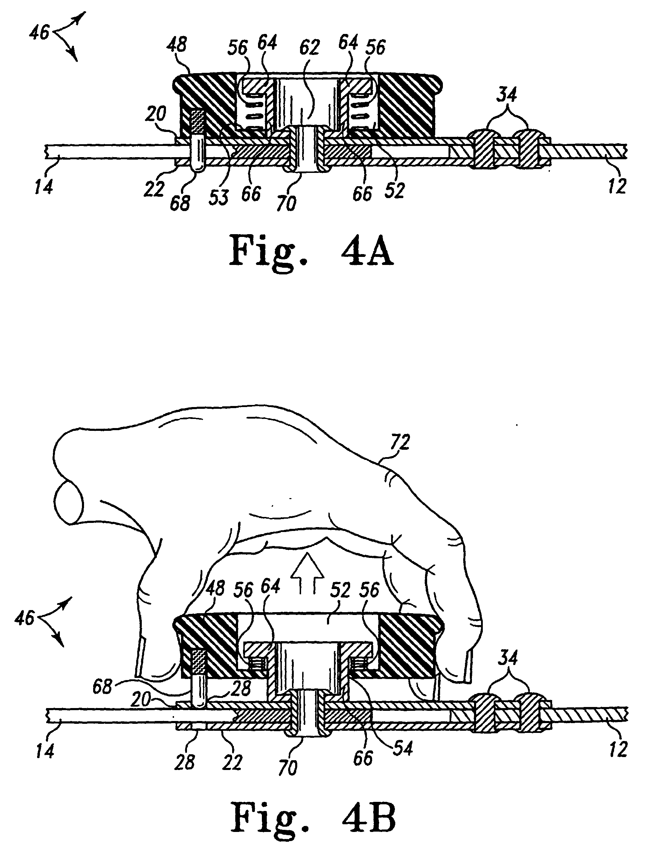

Referring to Figs. 4A and 4B, the operation of the axially actuated hinge assembly 16 can be seen. In the steady-state, engaged configuration, the spring 56, exerts opposing pressure against the retaining lip 64 and the bottom 53 of the chamber 52, thereby biassing the adjustment knob assembly 46 axially downward towards the base plates 20 and 22. This downward bias urges the stop pin 68 to captively engage one of the selected plurality of pairs of stop apertures 28a and 28b. When the lower strut 14 is pivoted toward the upper strut 12, the lower strut 14 abuts against the stop pin 68, which in the engaged configuration extends all the way down through the opposing base plates 20 and 22 to create a positive stop. This configuration could limit either the angle of extension or of flexion.

Referring to Fig. 4B, a hand 72 disengages the stop pin 68 by pulling the bezel 48 against the spring 56 axially away from the opposing base plates 20 and 22. By maintaining the upward force against the compressed spring 56, the bezel 48 may be rotated to select any of the plurality of pairs of circumferentially spaced stop apertures 28a and 28b (or positions) that correspond to the desired angle limit as clearly depicted on the index scale 50.

Through the use of an undepicted, and unconnected stop pin, angle limits on both extension and flexion can be set simultaneously. The undepicted stop pin could be manually inserted from the back of the inner base plate 22 to limit the movement in whatever direction, extension or flexion, that the adjustment assembly 46 described above is not limiting. Alternatively, in another undepicted embodiment, the adjustment assembly 46 can be adapted with an inner and an outer bezel. One of the two bezels could be adapted to control one stop pin as just described to limit, for example, the angle of extension. The other bezel could be adapted to work in opposition to the first bezel to limit the angle of movement in the other direction, for example flexion. This would be accomplished by reversing the direction of engagement and disengagement of the stop pin associated with the second bezel. Therefore, pushing down on the second bezel would disengage a stop pin from the back of the inner base plate 22, and releasing the second bezel would engage the stop pin.

## Claims

1. An orthopaedic joint brace comprising:
a first strut;
a second strut; and
a hinge assembly pivotally coupling the first strut with the second strut and defining an axis of rotation, in which a range of motion of the hinge assembly can be set selectively by an axially biassed stop.

2. A brace as claimed in claim 1, in which the stop comprises a retaining pin, and the hinge assembly includes a plurality of retaining apertures corresponding to particular ranges of motion and configured to receive the retaining pin.

3. A brace as claimed in claim 2, in which the plurality of retaining apertures are circumferentially space about the axis of rotation.

4. A brace as claimed in claim 3, in which the hinge assembly includes a pair of opposing plates, each plate having a plurality of apertures forming a plurality of opposing pairs of apertures that correspond to the plurality of retaining apertures for receiving the retaining pin.

5. The orthopaedic brace of claim 4, in which the pair of opposing plates are coupled to one of the first and second plates, and pivotally retain the other one of the first and second plates.

6. A brace as claimed in claim 2, in which the retaining pin is coupled to an axially displaceable and rotatable adjustment knob.

7. A brace as claimed in claim 2, in which the stop is axially biassed by a spring assembly.

8. A brace as claimed in claim 7, in which the spring assembly includes a spring retained by an adjustment knob that is rotatable to selectively position the retaining pin axially over one of the plurality of retaining apertures.

9. An orthopaedic brace comprising:
an upper strut;
a lower strut; and
a hinge assembly disposed between the struts and configured to allow movement of one of the struts about an axis corresponding to a joint axis, in which the hinge assembly can be set in any one of a plurality of positions corresponding to a plurality of ranges of motion of the struts about the axis, the hinge assembly having an axially actuated and rotatably mounted adjuster by which the hinge assembly can be set.

10. A brace as claimed in claim 9, in which the hinge assembly includes a plurality of retaining apertures corresponding to the plurality of positions, and the adjuster includes a retaining pin configured to selectively engage one of the plurality of retaining apertures to limit the range of motion of the hinge assembly.

11. A brace as claimed in claim 10, in which the plurality of retaining apertures are circumferentially spaced about the axis of rotation.

12. A brace as claimed in claim 11, in which the hinge assembly comprises opposing plates, each plate having a pivot aperture and a plurality of apertures forming a plurality of opposing pairs of apertures that correspond to the plurality of retaining apertures for receiving the retaining pin.

13. A brace as claimed in claim 12, in which the retaining pin is receivable into any one of the plurality of pairs of apertures from a front side of opposing plates and further comprising a second retaining pin which can be manually inserted from a back side of the opposing plates, in which one of the retaining pin and the second retaining pin limits flexion and the other of the retaining pin and the second retaining pin limits extension.

14. A brace as claimed in claim 10, in which the adjuster is axially biassed by a spring assembly.

15. A brace as claimed in claim 14, in which the spring assembly includes a spring retained by the adjuster that is rotatable to selectively position the retaining pin axially over one of the plurality of retaining apertures.

16. An orthopaedic brace comprising:
an upper strut;
a lower strut;
a hinge assembly disposed between the struts and configured to allow movement of one of the struts about an axis corresponding to a joint axis, the hinge assembly including opposing plates, each opposing plate containing a pivot aperture and a plurality of circumferential apertures forming pairs of retaining apertures, each pair of retaining apertures corresponding to a range of motion of the struts about the axis, the opposing plates rigidly connected to one of the upper and lower struts and configured to pivotally retain the other strut between them; and
an adjustment knob carried on the hinge assembly and including a retaining pin for engagement in a selective one of pairs of retaining apertures to select a range of motion of the struts about the axis.

17. A brace as claimed in claim 16, in which the adjustment knob can be actuated axially to select one of the pairs of retaining apertures.

18. A brace as claimed in claim 17, in which the adjustment knob is rotatably mounted to one of the opposing base plates.

19. A brace as claimed in claim 17, in which the adjustment knob comprises:
a bezel rigidly attached to the retaining pin; and
a spring assembly operable to bias against the bezel to maintain the bezel in a locked position in which the retaining pin is engaged with a selective one of the plurality of apertures, and configured to permit axial actuation of the bezel to disengage the retaining pin from the selective one of the plurality of apertures.

20. A brace as claimed in claim 19, in which the bezel is actuated axially outwardly to disengage the retaining pin.

21. A brace as claimed in claim 19, in which the bezel includes a degree scale corresponding to the pairs of retaining apertures to visually indicate a selected range of motion.

22. An orthopaedic brace comprising:
an upper strut;
a lower strut;
an axially actuated adjustment assembly having a first rotatable bezel with a first retaining pin fixed thereto, and a second rotatable bezel with a second retaining pin fixed thereto, the first and second rotatable bezels rotatable independently of each other;
a hinge assembly disposed between the upper and lower struts and providing movement of one of the upper and lower struts relative to the other of the upper and lower struts about an axis corresponding to a joint axis of a user, the hinge assembly including opposing plates each having a pivot aperture, a plurality of apertures, and rigidly coupled to one of the upper and lower struts and pivotally retaining the other of the upper and lower struts between them, the first and second rotatable bezels rotatably coupled to the hinge assembly; and
in which the axially actuated adjustment knob is configured to allow the first bezel and the first retaining pin to engage one of the plurality of pairs of apertures to set flexion, and to allow the second bezel and the second retaining pin to engage another one of the plurality of pairs of apertures to set extension.
